Europäisches Patentamt

European Patent Office

Office européen des brevets

(19)

(11) Veröffentlichungsnummer: **0 019 612**
**A1**

(12)

# EUROPÄISCHE PATENTANMELDUNG

(21) Anmeldenummer: 80890055.9

(22) Anmeldetag: 14.05.80

(51) Int. Cl.³: **A 61 F 1/02**
**A 61 F 1/12**

(30) Priorität: 17.05.79 AT 3654/79

(43) Veröffentlichungstag der Anmeldung:
26.11.80 Patentblatt 80/24

(84) Benannte Vertragsstaaten:
BE CH DE FR GB IT LI NL SE

(71) Anmelder: Grossberger, Gerhard
Degengasse 4/3
A-1160 Wien(AT)

(72) Erfinder: Grossberger, Gerhard
Degengasse 4/3
A-1160 Wien(AT)

(74) Vertreter: Köhler-Pavlik, Johann, Dipl.-Ing.
Margaretenplatz 5
A-1050 Wien(AT)

(54) Unterschenkelprothese.

(57) Unterschenkelprothese mit becherförmigem Stumpfbett (1) zur Aufnahme des Stumpfes und mit zwei seitlichen, das Kniegelenk übergreifenden Fortsätzen (2) die eine Fortsetzung des oberen Randes des Stumpfbettes (1) bilden. An der Innenseite jedes Fortsatzes (2) und gegebenenfalls an der Hinterseite der Innenwand des Stumpfbettes (1) ist je ein aufblasbares und entleerbares Luftkissen (3; 5) mit elastischer Wand vorgesehen, welches nach Einführen des Stumpfes durch eine handbetätigte, am Stumpfbett (1) vorgesehene Pumpeinrichtung aufgeblasen wird und einen einstellbaren Druck auf die Kondilen (oberhalb des Knies) und gegebenenfalls Wade zur Fixierung der Prothese ausübt. Der Luftdruck in den Luftkissen (3, 5) wird durch ein Ventilsystem gesteuert, dessen Betätigung ebenfalls von Hand aus erfolgt, so daß der Druck auf den Stumpf den jeweiligen Gegebenheiten angepaßt werden kann.

./...

EP 0 019 612 A1

FIG.2

0019612

## Unterschenkelprothese.

Die Erfindung betrifft eine Unterschenkelprothese mit becherförmigem Stumpfbett und mit seitlichen, das Kniegelenk übergreifenden Fortsätzen.

Bei Unterschenkelprothesen ohne Oberteil, d.h. Prothesen ohne mit dem Oberschenkel in Verbindung kommende Halteteile, war eine dauernde Verwendung der Prothese bei etwas komplizierten Stümpfen ohne Beschwerden und Schmerzen kaum möglich. Insbesondere war dies bei kurzen, konischen Unterschenkelstümpfen, bei Schwellstümpfen, bei sehr knöchernen Stümpfen und vor allem aber dann der Fall, wenn oberhalb des Kniegelenkes durch stark hervortretende Sehnen oder bei stark konischem Schenkel eine Belastung des Stumpfes nicht gut möglich ist.

Es sind schon Unterschenkelprothesen bekannt geworden, bei denen zur Aufnahme des Unterschenkelstumpfes ein doppelwandiger aufblasbarer Sack vorgesehen ist, welcher den Stumpf von allen Seiten her und von unten abstützt. Dieser Luftsack hatte aber nicht nur die Aufgabe, den Stumpf möglichst weich zu unterstützen, sondern mußte auch das Festhalten der Prothese am Stumpf bewirken. Zu diesem Zwecke mußte der Luftsack verhältnismäßig stark aufgepumpt werden und legt sich sehr fest gegen den Stumpf. Dieses feste Anliegen wurde aber, insbesondere bei höheren Temperaturen als sehr unangenehm empfunden. Trotz des festen Aufpumpens konnte aber anderseits niemals die für ein Gehen ohne Stock oder Krücke erforderliche und praktisch nur mit einem festen Stumpfbett erreichbare, möglichst starre Verbindung zwischen Stumpf und Prothese erzielt werden, weil die Innenwand des Luftsackes sich immer innerhalb der Außenwand desselben nach allen Seiten etwas bewegen konnte.

Die Erfindung hat sich daher zum Ziel gesetzt, Unterschenkelprothesen ohne Oberteil so zu verbessern, daß die angeführten Nachteile nicht mehr auftreten können und ein guter Halt der Prothese am Stumpf erzielt wird. Dies wird dadurch erreicht, daß bei einer Unterschenkelprothese der eingangs angegebenen Art erfindungsgemäß an der Innenseite jedes Fortsatzes und gegebenenfalls an der Hinterseite der Innenwand des Stumpfbettes je ein aufblasbares und entleerbares Luftkissen mit elastischer Wand vorgesehen ist, welches nach Einführen des Stumpfes aufgeblasen wird und einen einstellbaren Druck auf die Kondilen (oberhalb des Knies) und gegebenenfalls Wade zur Fixierung der Prothese ausübt.

Weitere Merkmale der Erfindung werden im folgenden an Hand der Zeichnungen erläutert, welche zwei Ausführungsbeispiele der erfindungsgemäßen Unterschenkelprothese darstellen. Hiebei ist

0019612

Figur 1 ein Längsschnitt der Prothese in Sagittalebene,

Figur 2 ist ein Schnitt durch die Prothese nach der Linie II-II in Figur 1,

Figur 3 ist ein vergrößerter Schnitt nach der Linie III-III in Figur 1 und

Figur 4 zeigt eine vereinfachte Ausführungsform der Prothese in vergrößerter Darstellung im Schnitt ähnlich wie Figur 1.

Der wesentliche Teil der Prothese, das Stumpfbett 1, ist genau nach dem Stumpf, der zusammen mit dem Knie und einem Teil des Oberschenkels mit strichpunktierten Linien angedeutet ist, mit Hilfe von Gipsabgüssen u. dgl. aus glasfaserverstärktem Kunststoff hergestellt. Das Stumpfbett 1 kann daher trotz geringer Wandstärke mit sehr hoher Festigkeit ausgeführt werden. In seinem oberen Teil ist das Stumpfbett 1 mit zwei seitlichen Fortsätzen 2 versehen, welche das Kniegelenk von beiden Seiten her überdecken. An der Innenseite jedes dieser Fortsätze 2 ist je ein Luftkissen 3 vorgesehen. Diese Luftkissen sind beispielsweise durch Ankleben einer Gummiplatte 4 hergestellt, deren Rand zum Teil den Rand des jeweiligen Fortsatzes 2 übergreift und andernteils zwischen zwei Schichten des das Stumpfbett 1 bildenden Materials eingelegt und verklebt ist. Die so gebildeten Luftkissen 3 sind im entleerten Zustand sehr flach, d.h. nur so dick wie die Gummiplatte selbst und hindern das Anlegen der Prothese in keiner Weise.

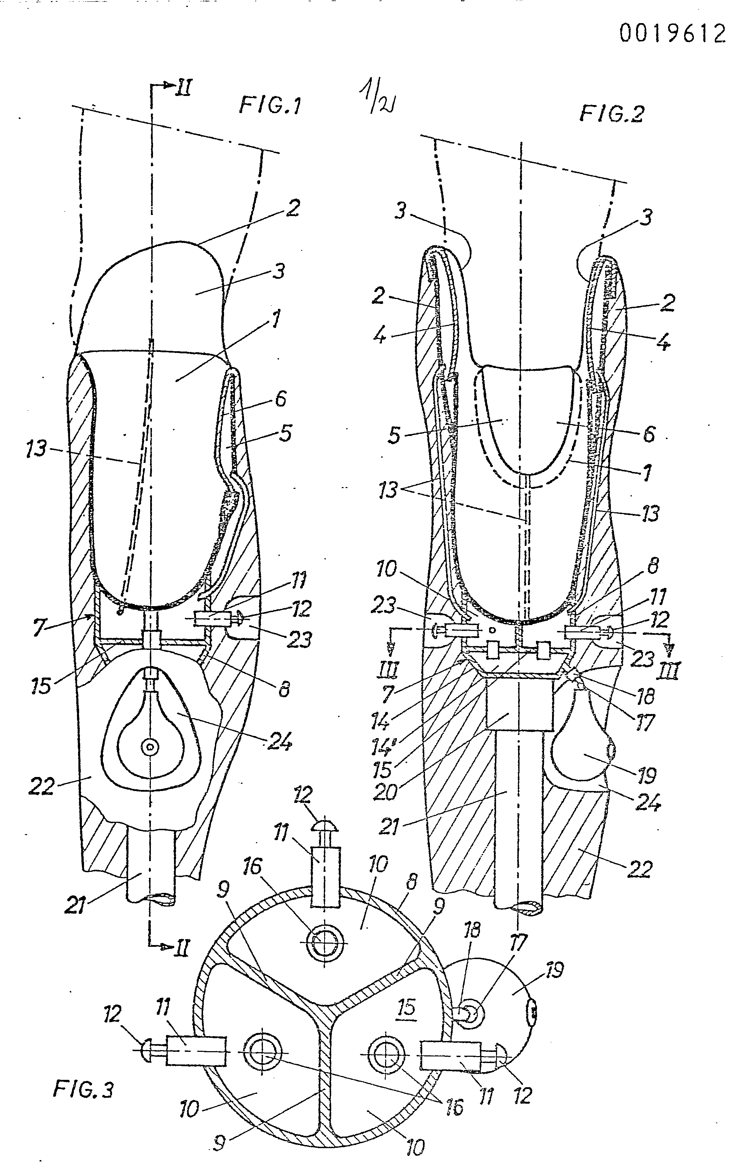

Knapp unterhalb des der Kniekehle zunächst liegenden Abschnittes des Stumpfbettes 1 ist bei der Ausbildungsart nach den Fig. 1 bis 3 ein drittes Luftkissen 5 angeordnet, welches in derselben Weise wie die zuvor erwähnten Luftkissen 3 hergestellt ist. Hiebei übergreift der obere Rand einer dünnen Weichgummiplatte 6 den Rand des Stumpfbettes 1 und ist an diesem Rand angeklebt; der übrige Randbereich der Weichgummiplatte 6 ist zwischen zwei Lagen des Materials des Stumpfbettes 1 eingelegt und dort durch Verkleben gesichert. Auch dieses Luftkissen ist sehr dünn oder flach und bildet im entleerten Zustand kein Hindernis für das Anlegen der Prothese.

Das Stumpfbett 1 ist an seinem unteren Teil mit einem dosenartigen hohlen Zwischenstück (Luftkissen-Adapter) 7 vorzugsweise durch Verkleben verbunden. Dieses Zwischenstück hat beispielsweise die Form eines flachen Zylinders, welcher auf einem umgekehrten Kegelstumpf aufsitzt. Der zylindrische Oberteil 8 des Zwischenstückes 7 ist durch radiale Zwischenwände 9 in so viele Kammern 10 unterteilt, als Luftkissen 3 und 5 - hier drei - vorhanden sind. Jede Kammer 10 ist mit einem Ablaßventil 11 versehen, welches im Normalzustand verschlossen ist und durch Drücken auf den Betätigungsknopf 12 geöffnet werden kann. Des weiteren geht von jeder Kammer 10 eine Leitung 13 aus, welche die jeweilige Kammer 10 mit dem ihr zugeordneten Luftkissen 3 und 5 unmittelbar verbindet.

Der zylindrische Oberteil 8 ist von kegelstumpfförmigen Unterteil 14 durch einen Zwischenboden 15 getrennt. In diesem Zwischenboden 15 sind drei Rückschlagventile 16 vorgesehen, deren jedes in eine der Kammern 10 führt. Der Innenraum des Unterteiles 14 bildet eine Druckkammer 14' und ist über ein kurzes Rohr 17, in welchem ein Rückschlagventil 18 untergebracht ist, mit einem kleinen Blasbalg 19 verbunden.

0019612

Am Boden des Unterteils 14 ist eine (nur angedeutete) Befestigungsvorrichtung 20, z.B. eine Schraubklemme od.dgl., zum Festhalten des den Prothesenunterteil bildenden Rohres 21 angebracht.

Die beschriebene Unterschenkelprothese wird durch eine Verkleidung 22 (sogenannte "Kosmetik"), z.B. aus Kunststoff, welche der Außenform des normalen menschlichen Unterschenkels entspricht, vervollständigt. Diese Verkleidung 22 ist mit Ausnehmungen 23 und 24 zur Aufnahme der Ablaßventile 11 mit Druckknopf 12 bzw. des Blasbalges 19 versehen.

Im folgenden wird der Gebrauch der erfindungsgemäßen Unterschenkelprothese erläutert.

Es wird vorausgesetzt, daß alle Luftkissen 3 und 5 durch Betätigen der Druckknöpfe 12 über die Leitungen 13 und die Kammern 10 entleert worden sind. Die Prothese kann in diesem Zustand leicht auf den Unterschenkelstumpf aufgeschoben werden. Sodann wird der Blasbalg 19 betätigt. Dadurch wird Luft in den Druckraum 14' im Unterteil 14 des Zwischenstückes 7 gedrückt. Durch den Überdruck in diesem Druckraum 14' öffnen die Rückschlagventile 16 und die Luft tritt in die Kammern 10 ein, von wo sie durch die Leitungen 13 in die drei Luftkissen 3 und 5 gelangt. Die Luftkissen 3 und 5 dehnen sich aus und legen sich von beiden Seiten her gegen das Kniegelenk bzw. von schräg-oben her gegen den Ansatz des Wadenmuskels unterhalb der Kniekehle. Der Blasbalg soll hiebei eher zuviel als zuwenig betätigt werden, um einen vorderhand etwas zu starken Innendruck in den Luftkissen 3 und 5 zu erzeugen. Der Träger der Prothese kann sodann durch kurzes Betätigen der einzelnen Druckknöpfe 12 aus jedem einzelnen der Luftkissen 3 und 5 verschieden viel Luft ablassen, um hiedurch in jedem dieser Kissen den seinem Empfinden nach richtigen Druck einzustellen, der beispielsweise zum Gehen als am besten geeignet angesehen wird.

Wird die Prothese zeitweilig nicht gebraucht oder soll sie nur wenig belastet werden oder besteht die Möglichkeit, den zum Halten der Prothese am Unterschenkel erforderlichen Druck zu mindern, etwa beim ruhigen Sitzen, beim Autofahren od.dgl., dann kann der Träger der Prothese durch Öffnen aller Ventile 11 die Luftkissen 3 und 5 weitgehend oder zur Gänze entleeren. Der Beinstumpf wird dadurch entlastet und kann sich von der vorhergegangenen Beanspruchung erholen.

Für den darauffolgenden Gebrauch der Prothese - etwa zum Aufstehen vom Sitz, zum Aussteigen aus dem Fahrzeug, zum Gehen - genügt es, durch Betätigen des Blasbalges 19 Luft in die Kissen 3 und 5 zu drücken und sodann durch Betätigen der Ventile 11, wie zuvor beschrieben, den richtigen Druck in den Luftkissen einzustellen.

Das Abnehmen der Prothese ist nach gänzlichem Entleeren aller Luftkissen mühelos möglich.

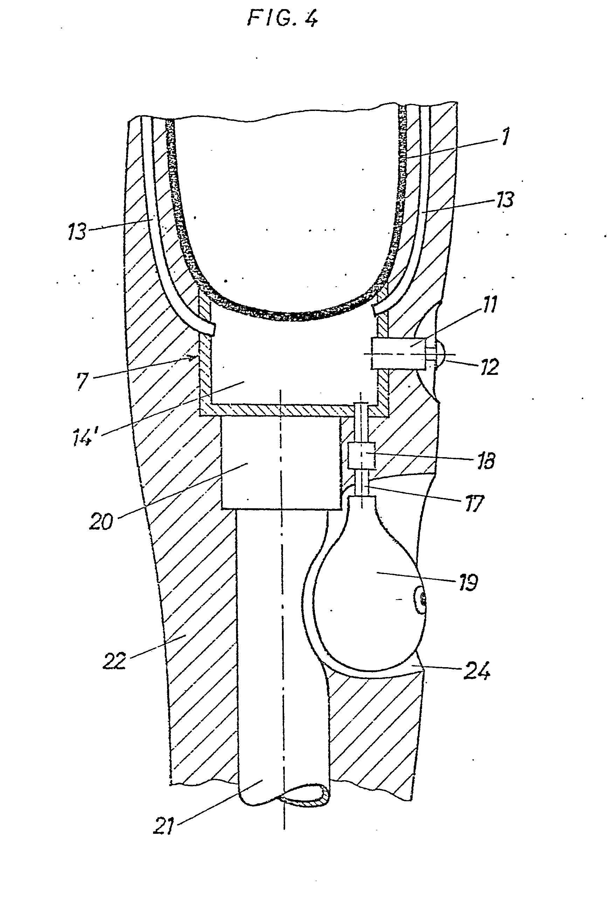

Die in Fig. 4 gezeigte, vereinfachte Ausführungsform der erfindungsgemäßen Prothese ist mit nur zwei Luftkissen versehen, d.h. das hintere Luftkissen (in den Fig. 1 und 2 mit 5 bezeichnet), ist nicht vorgesehen, und der Adapter 7 ist von vereinfachter Bauweise. Dieser Adapter 7 weist nur eine einzige Druckkammer 14' auf und hat keine Kammer 10. Die Leitungen 13 zu den seitlichen Luftkissen (nicht gezeigt) gehen daher unmittelbar von der Druckkammer 14' aus. Ebenso sind das kurze Rohr 17 mit Rückschlagventil 18 und Blasbalg 19 und das Ablaßventil 11 mit Druckknopf 12 an der Druckkammer 14' angebracht.

Die übrigen, in Fig. 4 eingetragenen Bezugszeichen haben die gleiche Bedeutung wie in den Fig. 1 und 2.

Diese einfachere Ausführungsform verursacht geringere Herstellungskosten und hat auch etwas geringeres Gewicht als die an Hand der Fig. 1 bis 3 beschriebene Ausbildungsart. Durch das Zusammenhängen der beiden Luftkissen 3 über die gemeinsame Druckkammer 14' stehen beide Luftkissen immer unter dem gleichen Innendruck und üben dieselbe Kraft auf das Kniegelenk aus. Eine gesonderte Einstellung des Druckes in jedem einzelnen der Luftkissen 3 ist daher nicht möglich.

Bei jeder Ausbildungsart der erfindungsgemäßen Prothese kann der Benützer durch Betätigen der Druckknöpfe 12 den Druck auf die beim Trägen der Prothese betroffenen Stellen des Beines nach eigenem Ermessen und nach eigener Erfahrung einstellen. Dadurch wird der bestmögliche Halt der Prothese am Unterschenkelstumpf erreicht und jede Pumpbewegung des Stumpfes gegenüber der Prothese (im Stumpfbett) wird vermieden. Das um ein geringes Ausmaß höhere Gewicht der erfindungsgemäßen Prothese - je nach Ausführungsart etwa 80 bis 100 g - gegenüber den Prothesen üblicher Bauart ist unwesentlich und wird vom Benützer kaum wahrgenommen.

Die erfindungsgemäße Bauweise ist bei jeder Unterschenkelamputation anwendbar. Das Luftkissen kann auch so ausgebildet sein, daß es durch ein anderes ausgetauscht werden kann.

Patentansprüche

1. Unterschenkelprothese mit becherförmigem Stumpfbett zur Aufnahme des Stumpfes und mit seitlichen, das Kniegelenk übergreifenden Fortsätzen, dadurch gekennzeichnet, daß an der Innenseite jedes Fortsatzes (2) und gegebenenfalls an der Hinterseite der Innenwand des Stumpfbettes (1) je ein aufblasbares und entleerbares Luftkissen (3; 5) mit elastischer Wand vorgesehen ist, welches nach Einführen des Stumpfes aufgeblasen wird und einen einstellbaren Druck auf die Kondilen (oberhalb des Knies) und gegebenenfalls Wade zur Fixierung der Prothese ausübt.

2. Prothese nach Patentanspruch 1, dadurch gekennzeichnet, daß zum Aufblasen aller Luftkissen (3; 5) ein Blasbalg (19) mit Rückschlagventil (18) vorgesehen ist.

3. Prothese nach Patentanspruch 2, dadurch gekennzeichnet, daß der Blasbalg (19) an eine unterhalb des Stumpfbettes (1) angeordnete Druckkammer (14') angeschlossen ist, mit welcher die Luftkissen (3; 5) in unmittelbarer Verbindung stehen.

4. Prothese nach Patentanspruch 3, dadurch gekennzeichnet, daß die Druckkammer (14') mit einem Ablaßventil (11) versehen ist.

5. Prothese nach Patentanspruch 3, dadurch gekennzeichnet, daß oberhalb der Druckkammer (14') Entlüftungskammern (10) vorgesehen sind, deren jede einerseits unmittelbar mit je einem der Luftkissen (3; 5) verbunden ist und anderseits über je ein Rückschlagventil (16) mit der Druckkammer (14') in Verbindung steht.

6. Prothese nach Patentanspruch 5, dadurch gekennzeichnet, daß jede Entlüftungskammer (10) mit einem eigenen Ablaßventil (10) versehen ist.

7. Prothese nach Patentanspruch 5, dadurch gekennzeichnet, daß die Druckkammer (14') und die Entlüftungskammern (10) zu einem dosenartigen Zwischenstück (Adapter 7) zusammengefaßt sind und durch eine Querwand (15) voneinander getrennt sind und daß die Entlüftungskammern (10) im wesentlichen kreissektorartige Form haben.

8. Prothese nach Patentanspruch 2 oder 7, dadurch gekennzeichnet, daß der Blasbalg (19) am unteren Ende des Zwischenstückes (7) nach unten ragend angebracht ist.

0019612

FIG.1    1/2    FIG.2

FIG.3

FIG. 4

**Europäisches Patentamt**

# EUROPÄISCHER RECHERCHENBERICHT

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| A | CH - A - 17 546 (N. FAARUP) <br> * Fig. 1 bis 3 * <br> -- | 1 | |
| A | US - A - 2 634 424 (T.C. O'GORMAN) <br> * Fig. 1, 2 * <br> -- | 1 | |
| A | US - A - 1 893 853 (A.E. TULLIS) <br> * Fig. 1 bis 3 * <br> ---- | 1 | RECHERCHIERTE SACHGEBIETE (Int. Cl.3) |

EPA Form 1503.2  06.78

0019612

# EUROPÄISCHER RECHERCHENBERICHT

Europäisches Patentamt

Nummer der Anmeldung

EP 80 89 0055.9

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US – A – 3 671 980 (L.F. BAIRD) <br> * Ansprüche 1 bis 3; Spalte 2, Zeilen 65 bis 75; Fig. 1, 2, 5 * <br> -- | 1,2,4 |
| | DE – C – 802 645 (R. HAINDL) <br> * Fig. 1 * <br> -- | 1 |
| | DE – A – 2 026 825 (COSMEVO LTD.) <br> * Ansprüche 1, 5; Seite 5, Zeilen 1 bis 3; Fig. 5 * <br> -- | 1,2 |
| | AT – B – 175 973 (M. ZIMMERMANN) <br> * Anspruch 1; Seite 1, Zeilen 29 bis 40; Fig. 1 * <br> -- | 1 |
| A | DE – C – 98 748 (N. FAARUP) <br> * Fig. 1 * <br> -- | 1 |
| A | DE – A1 – 2 526 574 (M.K. BONNER) <br> * Fig. 1 * <br> -- | 1 |
| A | DE – U – 1 736 231 (J. KÜPPERS) <br> * Ansprüche 1, 2; Fig. 1 bis 3 * <br> -- | 1 |

./..

**KLASSIFIKATION DER ANMELDUNG (Int.Cl.3)**

A 61 F 1/02
A 61 F 1/12

**RECHERCHIERTE SACHGEBIETE (Int. Cl.3)**

A 61 F 1/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 30-07-1980 | KANAL |

EPA form 1503.1 06.78